# EUROPÄISCHE PATENTSCHRIFT

(11) **EP 2 674 117 B1**
(45) Veröffentlichungstag und Bekanntmachung des Hinweises auf die Patenterteilung: **06.07.2016**
(21) Anmeldenummer: 13167574.6
(22) Anmeldetag: 14.05.2013
(51) Int. Cl.: A61B 17/29, A61B 17/32, F16D 3/16, F16D 3/20, F16D 3/207, F16C 1/04, F16C 1/10, A61B 17/00

(54) **Übertragungseinrichtung zum Übertragen einer Kraft und eines Drehmoments in einem medizinischen Instrument**
Transmission device for transmitting force and torque in a medical instrument
Dispositif de transmission pour la transmission d'une force et d'un couple dans un instrument médical

(30) Priorität: 13.06.2012 DE 102012105082
(43) Veröffentlichungstag der Anmeldung: 18.12.2013
(73) Patentinhaber: Karl Storz GmbH & Co. KG, 78532 Tuttlingen (DE)
(72) Erfinder: Kramer, Claus, 78532 Tuttlingen (DE); Stefan, Jochen, 78532 Tuttlingen (DE)

(56) Entgegenhaltungen:
- EP-A1- 2 255 734
- EP-A1- 2 287 478
- WO-A1-2010/112608
- DE-A1- 10 016 633
- US-A- 3 177 683
- US-A- 5 439 478
- US-A1- 2008 188 891
- US-A1- 2009 076 511
- US-A1- 2011 303 053

## Beschreibung

Die vorliegende Erfindung ist auf eine Übertragungseinrichtung zum Übertragen einer Kraft und eines Drehmoments zwischen einer Handhabungseinrichtung und einem Werkzeug eines medizinischen Instruments und auf ein Verfahren zum Herstellen einer Übertragungseinrichtung bezogen.

Die Erwartungen an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende, bei denen das Werkzeug um die Längsachse des Schafts rotierbar ist. Die Greif- oder Schneidefunktion und die Rotation des Werkzeugs sind beispielsweise mittels einer einzigen Übertragungsstange steuerbar, die Längskräfte und Drehmomente überträgt. Hinzu kommt in neuerer Zeit eine Abwinkelbarkeit des Schafts an einem Gelenk proximal des Werkzeugs. Dies setzt eine Flexibilität der Übertragungsstange im Bereich des Gelenks voraus.

In der DE 39 23 609 A1 ist eine Winkelantriebsvorrichtung mit einer Vielzahl von miteinander in Eingriff stehenden Bewegungsübertragungssegmenten beschrieben. Jedes Bewegungsübertragungssegment umfasst einen Kopfabschnitt und einen Fassungsabschnitt mit jeweils polygonalem Querschnitt. Der Kopfabschnitt eines Bewegungsübertragungssegments greift in einen Fassungsabschnitt eines benachbarten Bewegungsübertragungssegments ein. Die Winkelantriebsvorrichtung ermöglicht jedoch keine Übertragung von Zugkräften.

Die US 2009 0076511 A1 beschreibt einen Shaver, für insb. Bandscheiben. Dieser umfasst eine Übertragungseinrichtung zum Übertragen eines Drehmoments (zum Shaven) und einer (geringen) Kraft (, um den Shaver an zu shavendes Gewebe anzudrücken,) zu einem Werkzeug eines medizinischen Instruments, reamer. Die Übertragungseinrichtung ist mit Übertragungsgliedern offenbart, die miteinander gelenkig verbunden sind, wobei ein Kopplungsabschnitt eines ersten Übertragungsglieds in einem Kopplungsabschnitt eines benachbarten zweiten Übertragungsglieds derart formschlüssig gehalten ist, dass eine Rotation des ersten Übertragungsglieds um seine Längsachse eine Rotation des zweiten Übertragungsglieds um dessen Längsachse bewirkt und wobei der eine Kopplungsabschnitt konvex und das andere Konkav ist. US 2009 0076511 A1 offenbart eine Übertragungseinrichtung gemäß dem Oberbegriff des unabhängigen Anspruchs 1.
US20110303053A1 stellt eine Werkzeugverlängerung für einen Steckschlüssel vor. Diese enthält eine Reihe Gelenken mit Vierkant-Elementen mit Paarungen von männlichen, Außenmehrkant-, und weiblichen, Innenmehrkant-Enden, so dass die Gliederkette in einer gekrümmten Bahn verlaufen kann.

In der Druckschrift US3177683A ist eine Kabelkonfektion gezeigt, die mittels Kugelgelenkaufbau flexibel dargestellt ist, um auch einen gekrümmten Verlauf zu gewährleisten. Fixierbar sind die Kugelgelenke dabei mittels "clips".

Die DE10016633A1 zeigt eine Welle, deren mehrere Wellenglieder flexibel gestaltet sind, indem sie derart aneinander anliegen, dass sie jeweils Ausnehmungen und dazu korrespondierend Ansätze aufweisen, die mäander-artig in einander eingreifen.

In der Patentanmeldung EP2287478A1 ist ein Element beansprucht, das über eine Abwinklung eines Schafts eines endoskopischen Instruments Zug-, Druck, wie auch Torsionskräfte übertragen kann. Dies erfolgt gemäß Lehre der Schrift, indem eine Konstruktion Balgen für die Torsionskräfte mit angeschrägten Beilagscheiben für die Druckkräfte und einem Kabel für die Zugkräfte verbindet..

Die US20080188891 A1 zeigt ein medizinisches Greif-Instrument mit einem Handgriff, einem Schaft, zwei Greifbacken, von denen wenigstens eines schwenkbar zum Schaft bzw. der anderen Backe ist, wobei der Schaft abschnittsweise abwinkelbar ist, und die Maulteile im geschlossenen Zustand fixierbar sind.

In US 5439478 A ist ein lenkbares flexibles mikrochirurgisches Instrument gezeigt, das einen drehbaren Gabelkopf aufweist. Das Instrument umfasst eine flexible Spule, einen flexiblen Zugdraht durch die Spule, einen Gabelkopf und ein Werkzeug, wobei der Gabelkopf drehbar mit dem distalen Ende der Spule und das Werkzeug mit dem distalen Ende des Zugdrahts gekoppelt sind.Eine Aufgabe der vorliegenden Erfindung besteht darin, eine verbesserte Übertragungseinrichtung zum Übertragen einer Kraft und eines Drehmoments zwischen einer Handhabungseinrichtung und einem Werkzeug eines medizinischen Instruments, ein verbessertes Werkzeug für ein medizinisches Instrument, ein verbessertes medizinisches Instrument und ein verbessertes Verfahren zum Herstellen einer Übertragungseinrichtung zu schaffen.

Diese Aufgabe wird durch die Gegenstände der unabhängigen Ansprüche gelöst.

Weiterbildungen sind in den abhängigen Ansprüchen angegeben.

Eine Übertragungseinrichtung zum Übertragen einer Kraft und eines Drehmoments zwischen einer Handhabungseinrichtung und einem Werkzeug eines medizinischen Instruments umfasst miteinander gelenkig verbundene Übertragungsglieder, wobei jeweils ein konvexer Kopplungsabschnitt eines ersten Übertragungsglieds in einem konkaven Kopplungsabschnitts eines benachbarten zweiten Übertragungsglieds derart formschlüssig gehalten ist, dass eine Rotation des ersten Übertragungsglieds um seine Längsachse eine Rotation des zweiten Übertragungsglieds um seine Längsachse bewirkt, wobei jeweils ein konvexer Kopplungsabschnitt eines ersten Übertragungsglieds in einem konkaven Kopplungsabschnitt eines benachbarten zweiten Übertragungsglieds derart formschlüssig gehalten ist, dass eine Zugkraft zwischen dem ersten Übertragungsglied und dem zweiten Übertragungsglied übertragbar ist.

Die Übertragungseinrichtung ist insbesondere für ein medizinisches Instrument für mikroinvasiv-chirurgische Eingriffe ausgebildet und weist einen Schaft zwischen Handhabungs einrichtung und Werkzeug auf. Die Übertragungseinrichtung ist zum Übertragen eines Drehmoments und zum Übertragen von sowohl Druck- als auch Zugkräften in dem Schaft vorgesehen und ausgebildet. Die gelenkig verbundenen Übertragungsglieder sind insbesondere dazu vorgesehen und ausgebildet, in einem gekrümmten oder krümmbaren bzw. flexiblen oder elastischen Bereich des Schafts angeordnet zu werden.

Damit eine Rotation eines ersten Übertragungsglieds um seine Längsachse eine Rotation eines zweiten Übertragungsglieds um seine Längsachse bewirkt, sind die Übertragungsglieder insbesondere torsionssteif miteinander gekoppelt bzw. in einer zur Übertragung von Drehmomenten geeigneten Weise miteinander gekoppelt. Insbesondere weisen der konvexe Kopplungsabschnitt eines Übertragungsglieds und der konkave Kopplungsabschnitt eines benachbarten Übertragungsglieds jeweils korrespondierende polygonale Querschnitte auf, beispielsweise sechseckige Querschnitte.

Mitteils einer Übertragungseinrichtung mit den beschriebenen Merkmalen können nicht nur Drehmomente und Druckkräfte, sondern auch Zugkräfte übertragen werden. Dies kann beispielsweise eine Steuerung einer Rotation und sowohl eines Öffnens als auch eines Schließens von Maulteilen eines Werkzeugs am distalen Ende eines krümmbaren oder abwinkelbaren Schafts ermöglichen, wie es von medizinischen Instrumenten mit starrem geraden Schaft bereits bekannt ist.

Bei einer Übertragungseinrichtung, wie sie hier beschrieben ist, sind am konvexen Kopplungsabschnitt eines ersten Übertragungsglieds ein erster Haltebereich und am konkaven Kopplungsabschnitt eines benachbarten zweiten Übertragungsglieds ein zweiter Haltebereich vorgesehen und der erste Haltebereich und der zweite Haltebereich formschlüssig miteinander verbunden, um eine Zugkraft zwischen dem ersten Übertragungsglied und dem zweiten Übertragungsglied aufzunehmen.

Bei einer Übertragungseinrichtung, wie sie hier beschrieben ist, ist bezogen auf eine Schnittebene, die die Längsachse des ersten Übertragungsglieds enthält, der erste Haltebereich zu einem konkaven Bereich der Kontur des Übertragungsglieds benachbart, wobei der zweite Haltebereich eine im Wesentlichen konvexe Kontur aufweist.

Die Kontur des ersten Haltebereichs selbst in der Schnittebene ist insbesondere konvex. Der zweite Haltebereich kann eine Gleitfläche aufweisen, deren Gestalt an die Gestalt der Oberfläche des ersten Haltebereichs angepasst ist bzw. zu dieser korrespondiert. Die im Wesentlichen konvexe Kontur des zweiten Haltebereichs kann im Bereich der Gleitfläche konkav sein.

Bei einer Übertragungseinrichtung, wie sie hier beschrieben ist, wird der erste Haltebereich am ersten Übertragungsglied durch den Rand einer Nut gebildet, wobei der zweite Haltebereich am zweiten Übertragungsglied die Gestalt eines Kragens, der in die Nut am ersten Übertragungsglied eingreift, aufweist.

Die Nut ist insbesondere eine umlaufende, den gesamten Umfang des ersten Übertragungsglieds (insbesondere in einer Ebene senkrecht zu dessen Längsachse) einnehmende, nach radial außen offene Nut. Die Nut bildet insbesondere den oben genannten konkaven Bereich der Kontur des ersten Übertragungsglieds in der die Längsachse enthaltenen Schnittebene. Der erste Haltebereich wird insbesondere durch einen schulterförmigen Bereich am ersten Übertragungsglied gebildet, der gleichzeitig ein Randbereich oder eine Flanke der Nut ist. Der zweite Haltebereich am zweiten Übertragungsglied weist insbesondere die Gestalt eines umlaufenden bzw. im Wesentlichen geschlossenen und nach radial innen ragenden Kragens auf. Der Kragen weist insbesondere die Gestalt eines vollständigen oder fast vollständigen Kreisrings auf.

Bei einer Übertragungseinrichtung, wie sie hier beschrieben ist, ist der erste Haltebereich insbesondere durch eine Verjüngung des ersten Übertragungsglieds an einer von dem zweiten Übertragungsglied abgewandten Seite des konvexen Kopplungsabschnitts des ersten Übertragungsglieds gebildet.

Bei einer Übertragungseinrichtung, wie sie hier beschrieben ist, bildet eine Hülse, die den konkaven Kopplungsabschnitt umgibt, den nach radial innen ragenden Kragen.

Den nach radial innen ragenden Kragen an einer Hülse vorzusehen, kann eine kostengünstige Fertigung ermöglichen, bei der beispielsweise der konvexe Kopplungsabschnitt für ein erstes Übertragungsglied zunächst in der Hülse angeordnet und dann mit dem ersten Übertragungsglied gefügt wird, wonach die Hülse mit dem zweiten Übertragungsglied gefügt wird.

Bei einer Übertragungseinrichtung, wie sie hier beschrieben ist, umfasst die Hülse insbesondere einen Schlitz, der parallel zur Längsachse des zweiten Übertragungsglieds ist.

Bei einer Übertragungseinrichtung, wie sie hier beschrieben ist, ist der erste Haltebereich am ersten Übertragungsglied insbesondere durch eine Verjüngung des ersten Übertragungsglieds an einer von dem zweiten Übertragungsglied abgewandten Seite des konvexen Kopplungsabschnitts gebildet, wobei der Schlitz in der Hülse eine Breite aufweist, die nicht kleiner ist als eine minimale Breite der Verjüngung.

Der konvexe Kopplungsabschnitt kann einstückig mit anderen Bereichen des ersten Übertragungsglieds gebildet werden, beispielsweise mittels spanabhebender Verfahren. Danach kann die Verjüngung zwischen dem konvexen Kopplungsabschnitt und dem Rest des ersten Übertragungsglieds durch den Schlitz der Hülse hindurchgeführt werden, bis der Kragen an der Hülse den konvexen Kopplungsabschnitt hintergreift. Danach kann die Hülse mit dem zweiten Übertragungsglied gefügt werden. Die Verjüngung weist insbesondere die Gestalt eines Halses bzw. einer Taille auf.

Ein Werkzeug für ein medizinisches Instrument umfasst eine Übertragungseinrichtung, wie sie hier beschrieben ist.

Ein medizinisches Instrument umfasst eine Übertragungseinrichtung, wie sie hier beschrieben ist.

Bei einem Verfahren zum Herstellen einer Übertragungseinrichtung werden mehrere Übertragungsglieder mit jeweils zwei Kopplungsabschnitten bereitgestellt, wobei eines der mehreren Übertragungsglieder eine Hülse mit einem sich vom ersten Ende zum zweiten Ende der Hülse erstreckenden Schlitz und einen nach radial innen ragenden Kragen am zweiten Ende der Hülse aufweist. Eine Verjüngung an einem konvexen Kopplungsabschnitt an einem ersten Übertragungsglied wird vom ersten Ende durch den Schlitz bis zum zweiten Ende der Hülse hindurchgeführt. Ein konkaver Kopplungsabschnitt an einem zweiten Übertragungsglied wird in die Hülse eingeführt, wobei der konvexe Kopplungsabschnitt am ersten Übertragungsglied in den konkaven Kopplungsabschnitt am zweiten Übertragungsglied eingeführt wird. Die Hülse wird mit dem zweiten Übertragungsglied gefügt.

Das beschriebene Verfahren kann eine einfache und kostengünstige Herstellung einer Übertragungseinrichtung, insbesondere einer Übertragungseinrichtung mit den oben beschriebenen Merkmalen, ermöglichen. Insbesondere sind alle Übertragungsglieder gleich und weisen jeweils eine Hülse mit einem sich vom ersten Ende zum zweiten Ende der Hülse erstreckenden Schlitz auf. Alternativ sind zwei oder mehr verschiedene Typen von Übertragungsgliedern vorgesehen, wobei jedes einzelne Übertragungsglied an jedem Ende, an einem Ende oder an keinem Ende eine Hülse mit einem nach radial innen ragenden Kragen aufweisen kann.

### Kurzbeschreibung der Figuren

Nachfolgend werden Ausführungsformen anhand der beigefügten Figuren näher erläutert.

Es zeigen:
- Figur 1: eine schematische Darstellung eines medizinischen Instruments;
- Figur 2: eine schematische Schnittdarstellung eines flexiblen Abschnitts einer Übertragungseinrichtung;
- Figur 3: eine weitere schematische Schnittdarstellung des flexiblen Abschnitts aus Figur 2;
- Figur 4: eine weitere schematische Schnittdarstellung von Kopplungsgliedern aus den Figuren 2 und 3;
- Figur 5: eine weitere schematische Schnittdarstellung der Kopplungsglieder aus Figur 4;
- Figur 6: eine schematische Schnittdarstellung eines weiteren flexiblen Abschnitts einer Übertragungseinrichtung;
- Figur 7: ein schematisches Flussdiagramm.

### Beschreibung der Ausführungsformen

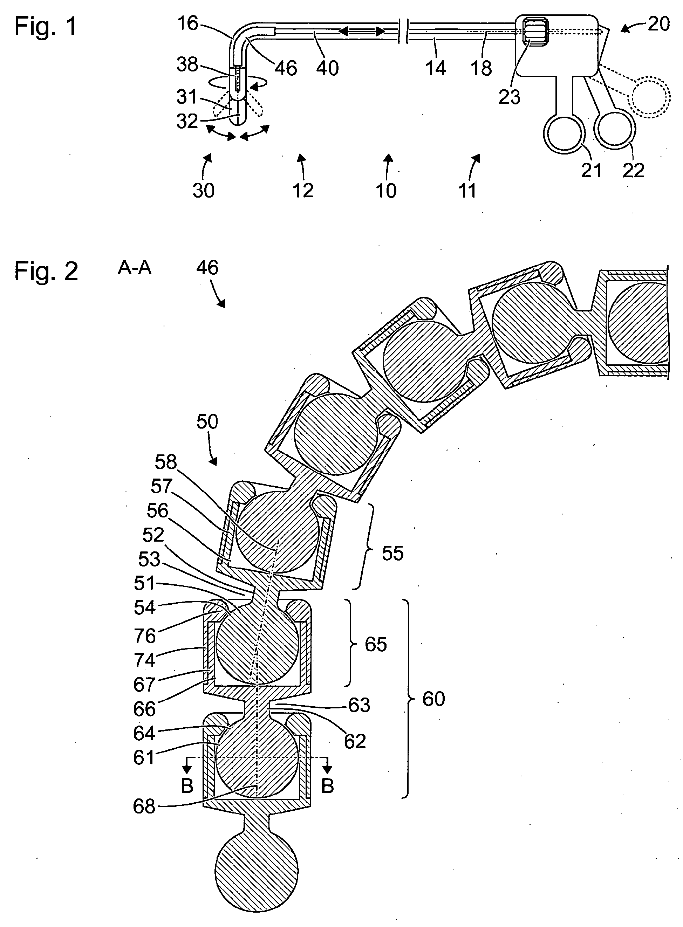

Figur 1 zeigt eine schematische Darstellung eines medizinischen Instruments 10 mit einem proximalen Ende 11 und einem distalen Ende 12, zwischen denen sich ein Schaft 14 mit einem Gelenk 16 erstreckt. Der Schaft 14 ist proximal des Gelenks 16 insbesondere starr und gerade und weist eine Längsachse 18 auf. Das Gelenk 16 wird durch einen flexiblen oder elastischen Abschnitt des Schafts 14 oder durch eine gelenkige Verbindung zweier jeweils starrer Abschnitte des Schafts 14 gebildet.

Am proximalen Ende 11 weist das medizinische Instrument 10 eine Handhabungseinrichtung 20 mit einem feststehenden Griffteil 21 und einem bewegbaren Griffteil 22 auf. Der bewegbare Griffteil 22 ist insbesondere um eine Schwenkachse senkrecht zur Zeichenebene der Figur 1 schwenkbar. Eine erste Position des bewegbaren Griffteils 22 ist in Figur 1 mit durchgezogenen Linien dargestellt. Eine zweite Position des bewegbaren Griffteils 22 ist in Figur 1 mit gestrichelten Linien dargestellt. Ferner weist die Handhabungseinrichtung 20 ein Drehrad 23 auf, das um die Längsachse 18 des Schafts 14 rotierbar ist.

Am distalen Ende 12 des medizinischen Instruments 10 ist ein Werkzeug 30 mit zwei schwenkbaren Maulteilen 31, 32 vorgesehen. In Figur 1 ist durch zwei Pfeile angedeutet, dass die schwenkbaren Maulteile 31, 32 jeweils um eine zugeordnete Schwenkachse senkrecht zur Zeichenebene der Figur 1 zwischen geschlossenen Positionen und offenen Positionen schwenkbar sind. Die geschlossenen Positionen sind in Figur 1 mit durchgezogenen Linien dargestellt, die offenen Positionen sind in Figur 1 mit gestrichelten Linien dargestellt. Ferner ist das Werkzeug 30 mit den Maulteilen 31, 32 um eine Rotationsachse 38 rotierbar, die insbesondere die Längsachse des Werkzeugs 30 ist.

Die Handhabungseinrichtung 20 am proximalen Ende 11 und das Werkzeug 30 am distalen Ende 12 des medizinischen Instruments 10 sind mittels einer Übertragungseinrichtung 40, insbesondere einer Übertragungsstange, im Schaft 14 gekoppelt. Die Übertragungseinrichtung 40 umfasst einen flexiblen Abschnitt 46, der unten mit Bezug auf die Figuren 2 bis 6 dargestellt ist. Die Übertragungseinrichtung 40 koppelt insbesondere den bewegbaren Griffteil 22 der Handhabungseinrichtung 20 und das Drehrad 23 an der Handhabungseinrichtung 20 einerseits mit dem Werkzeug 30 andererseits.

Insbesondere wird eine manuelle Bewegung des bewegbaren Griffteils 22 an der Handhabungseinrichtung 20 durch eine axiale Bewegung der Übertragungseinrichtung 40 parallel zur Längsachse 18 des medizinischen Instruments 10 und distal des flexiblen Abschnitts 46 parallel zu der Rotationsachse 38 des Werkzeugs 30 zu den Maulteilen 31, 32 übertragen und bewirkt öffnende oder schließende Schwenkbewegen der Maulteile 31, 32. Eine Rotation des Drehrads 23 um die Längsachse 18 wird durch eine Rotation der Übertragungseinrichtung 40 um die Längsachse 18 und distal des flexiblen Abschnitts 46 um die Längsachse 38 des Werkzeugs 30 zu dem Werkzeug 30 übertragen und bewirkt eine Rotation des Werkzeugs 30 um seine Rotationsachse 38.

Für eine spiel- und reibungsarme Übertragung von Längskräften und Drehmomenten ist die Übertragungseinrichtung 40 im Schaft 14 spiel- und reibungsarm gelagert und hinsichtlich Druck- und Zugkräften steif bzw. unelastisch und ferner torsionssteif bzw. steif hinsichtlich einer Torsion um ihre Längsachse ausgebildet. Dies gilt insbesondere auch für den flexiblen Abschnitt 46.

Figur 2 zeigt eine schematische Schnittdarstellung eines Ausführungsbeispiels des flexiblen Abschnitts 46 der Übertragungseinrichtung 40 aus Figur 1. Die Schnittebene A-A der Figur 2 ist parallel zur Zeichenebene der Figur 1 und enthält insbesondere die Längsachse 18 des Schafts 14 und die Längsachse 38 des Werkzeugs 30. Der flexible Abschnitt 46 umfasst eine Mehrzahl baugleicher Übertragungsglieder 50, 60, deren Längsachsen 58, 68 ebenfalls in der Schnittebene der A-A der Figur 2 liegen.

Jedes Übertragungsglied 50, 60 umfasst einen konvexen Kopplungsabschnitt 51, 61 und einen konkaven Kopplungsabschnitt 55, 65. In der Schnittebene A-A der Figur 2 weisen die konvexen Kopplungsabschnitte 51, 61 jeweils einen im Wesentlichen kreisförmigen Querschnitt auf. Jedes Übertragungsglied 50, 60 weist zwischen dem konvexen Kopplungsabschnitt 51, 61 und dem konkaven Kopplungsabschnitt 55, 65 eine Verjüngung bzw. einen Hals 52, 62 bzw. eine umlaufende Nut 53, 63 auf.

Die konkaven Kopplungsabschnitte 55, 65 umfassen Ausnehmungen 56, 66, die in der Schnittebene A-A jeweils einen im Wesentlichen rechteckigen Querschnitt aufweisen und von einer Wand 57, 67 umgeben sind. Jeder konkave Kopplungsabschnitt 55, 65 umfasst ferner eine Hülse 70. Jede Hülse 70 weist eine im Wesentlichen zylindrische Wand 74 auf, die die Wand 57, 67 um die Ausnehmung 56, 66 mantelförmig umgibt. Die Hülse 70 weist ferner einen nach radial innen ragenden Kragen 76 auf. Die Hülsen 70 sind mit den konkaven Kopplungsabschnitten 55, 65 gefügt, insbesondere geschweißt.

Es greift jeweils der konvexe Kopplungsabschnitt 51 an einem ersten Übertragungsglied 50 in den konkaven Kopplungsabschnitt 65 an einem zweiten, benachbarten Übertragungsglied 60 ein. Der Kragen 76 an der Hülse 70 des konkaven Kopplungsabschnitts 65 des zweiten Übertragungsglieds 60 greift teilweise in die Nut 53 am ersten Übertragungsglied 50 ein und liegt an einem schulterförmigen Bereich 54 am konvexen Kopplungsabschnitt 51 des ersten Übertragungsglieds 50 an. Die zum Anliegen an dem schulterförmigen Bereich 54 am konvexen Kopplungsabschnitt 51 des ersten Übertragungsglieds 50 vorgesehene Gleitfläche am Kragen 76 weist entsprechend dem in der Schnittebene A-A im Wesentlichen kugelförmigen Querschnitt des konvexen Kopplungsabschnitts 51 insbesondere die Gestalt eines ringförmigen Ausschnitts einer Kugeloberfläche auf.

Durch den zumindest teilweisen Eingriff des Kragens 76 eines Übertragungsglieds 60 in die Nut 53 am benachbarten Übertragungsglied 50 bzw. durch das Anliegen des Kragens 76 eines Übertragungsglieds 60 am schulterförmigen Bereich 54 am konvexen Kopplungsabschnitt 51 des benachbarten Übertragungsglieds 50 sind nicht nur Druckkräfte, sondern auch Zugkräfte zwischen den Übertragungsgliedern 50, 60 übertragbar. Damit sind auch durch den gesamten flexiblen Abschnitt 46 der Übertragungseinrichtung 40 Zugkräfte übertragbar. Die Darstellung in Figur 2 zeigt, dass die formschlüssige Kopplung der Übertragungsglieder 50, 60 gelenkig ist. Deshalb können die Längsachsen 58, 68 benachbarter Übertragungsglieder 50, 60 innerhalb eines vorbestimmten Winkelintervalls relativ zueinander gekippt werden.

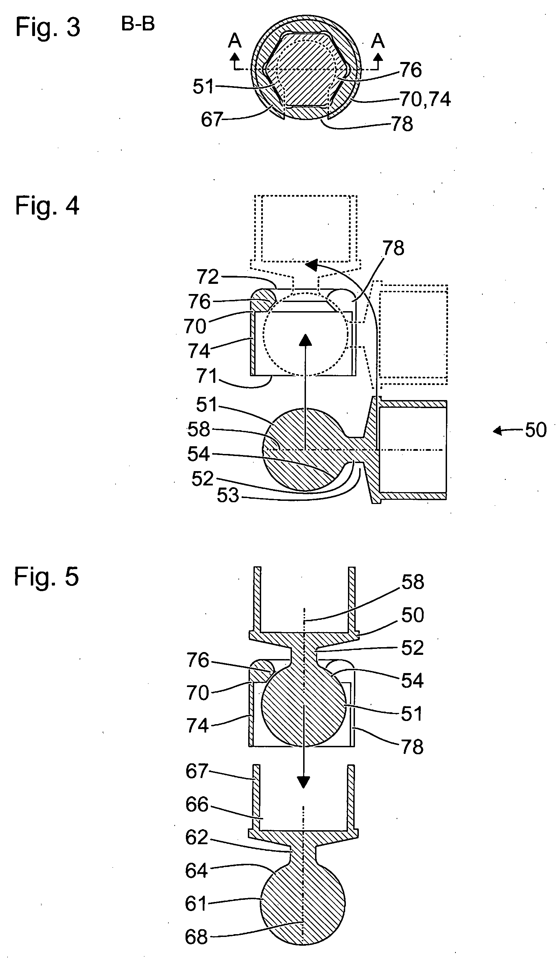

Figur 3 zeigt eine weitere schematische Schnittdarstellung des flexiblen Abschnitts 46 aus Figur 2. Die Lage der in Figur 3 dargestellten Schnittebene B-B senkrecht zur Längsachse 68 eines Übertragungsglieds 60 ist in Figur 2 angedeutet. Die Lage der in Figur 2 dargestellten Schnittebene A-A ist in Figur 3 angedeutet.

In der Schnittebene B-B weisen sowohl der konvexe Kopplungsabschnitt 51 als auch die innere Oberfläche der Wand 67 jeweils einen im Wesentlichen sechseckigen Querschnitt auf. Die einander korrespondierenden, im Wesentlichen sechseckigen Querschnitte des konvexen Kopplungsabschnittes 51 des ersten Übertragungsglieds 50 und der Wand 67 des konkaven Kopplungsabschnitts 65 des zweiten Übertragungsglieds 60 bewirken eine formschlüssige Kopplung der Übertragungsglieder 50, 60, die eine Übertragung eines Drehmoments zwischen den Übertragungsgliedern 50, 60 ermöglicht.

Die äußere Oberfläche der Wand 67 und die Wand 74 der Hülse 70 weisen jeweils im Wesentlichen die Gestalt eines Kreiszylindermantels auf. Abweichen davon weist die Hülse 70 einen Schlitz 78 auf, dessen Breite in der Schnittebene B-B mindestens so groß wie der Durchmesser des Halses 52 (vgl. Figur 2) ist. In gestrichelter Linie ist die radial innere Kontur des Kragens 76 der Hülse 70 sowie der Fortsetzung des Schlitzes 78 in den Kragen 76 hinein dargestellt. Der Kragen 76 weist also nicht die Gestalt eines geschlossenen Kreisrings sondern abweichend davon eine dem Schlitz 78 entsprechende Lücke auf.

Figur 4 zeigt eine schematische Schnittdarstellung eines ersten Übertragungsglieds 50 und einer Hülse 70 eines zweiten Übertragungsglieds, dessen weitere Bestandteile in Figur 4 nicht sichtbar sind. Die Schnittebene der Figur 4 ist senkrecht zur Schnittebene A-A der Figur 2 und senkrecht zur Schnittebene B-B der Figur 3 und enthält die Längsachse 58 des Übertragungsglieds 50. Die Schnittebene der Figur 4 verläuft insbesondere in der Mitte des Schlitzes 78 in der Hülse 70 und parallel zu dessen Rändern.

Das Übertragungsglied 50 ist mit durchgezogenen Linien und schraffierter Querschnittsfläche in einer anfänglichen Position gezeigt, in der die Längsachse 58 des Übertragungsglieds 50 senkrecht zur Längsachse der Hülse 70 ist. Von dieser anfänglichen Position aus wird der konvexe Kopplungsabschnitt 51 des Übertragungsglieds 50 mit der durch den geraden Pfeil angedeuteten reinen Translationsbewegung von deren ersten Ende 71 aus in die Hülse 70 eingeführt bis der konvexe Kopplungsabschnitt 51 des Übertragungsglieds 50 am Kragen 76 der Hülse 70 im Wesentlichen anliegt und der Hals 52 des Übertragungsglieds 50 in dem Schlitz 78 in der Hülse 70 angeordnet ist. Die so erreichte Konfiguration ist durch gestrichelte Konturen angedeutet.

Wie in Figur 4 durch einen gekrümmten Pfeil angedeutet ist, wird das Übertragungsglied 50 danach um eine Achse senkrecht zur Zeichenebene der Figur 4 und dabei um den konvexen Kopplungsabschnitt 51 geschwenkt bis die Längsachse des Übertragungsglieds 50 im Wesentlichen parallel zur Längsachse der Hülse 70 ist. Die so erreichte Konfiguration ist ebenfalls durch gestrichelte Konturen angedeutet. In dieser Konfiguration ist der Hals 52 des Übertragungsglieds 50 nicht mehr im Schlitz 78 in der Hülse 70, sondern im Wesentlichen in der Mitte des Kragens 76 am zweiten Ende 72 der Hülse 70 angeordnet.

Figur 5 zeigte eine weitere schematische Schnittdarstellung des Übertragungsglieds 50 und der Hülse 70 aus Figur 4 in der Konfiguration, die am Ende der anhand der Figur 4 beschriebenen Bewegung vorliegt. Das Übertragungsglied 50 und die Hülse 70 sind zusammen mit einem weiteren Übertragungsglied 60 dargestellt. Durch einen geraden Pfeil ist eine Bewegung des Übertragungsglieds 50 zusammen mit der Hülse 70 relativ zum weiteren Übertragungsglied 60 angedeutet. Bei dieser Bewegung wird der konvexe Kopplungsabschnitt 51 des Übertragungsglieds 50 in die Ausnehmung 66 am weiteren Übertragungsglied 60 eingeführt. Gleichzeitig wird die Hülse 70 über die Wand 67 um die Ausnehmung 66 am weiteren Übertragungsglied 60 gestülpt bzw. die Wand 67 in die Hülse 70 eingeführt. Wenn die in Figur 2 dargestellte Konfiguration erreicht ist, werden die Hülse 70 und das weitere Übertragungsglied 60 gefügt, insbesondere durch Schweißen, Löten oder Kleben.

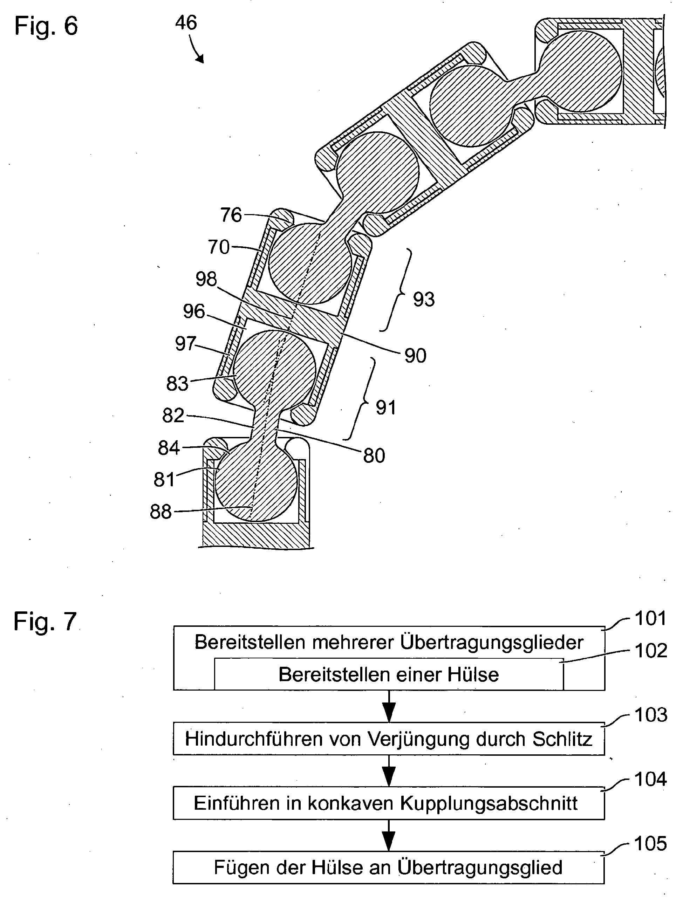

Figur 6 zeigt eine schematische Schnittdarstellung eines weiteren Ausführungsbeispiels des flexiblen Abschnitts 46 der Übertragungseinrichtung 40 aus Figur 1. Die Schnittebene der Figur 6 entspricht der Schnittebene der Figur 2.

Das Ausführungsbeispiel der Figur 6 unterscheidet sich vom Ausführungsbeispiel der Figuren 2 bis 5 dadurch, dass nicht alle Übertragungsglieder gleich sind. Stattdessen sind Übertragungsglieder 80, 90 zweier unterschiedlicher Typen bzw. Bauarten vorgesehen, die abwechselnd angeordnet sind. Ein Übertragungsglied 80 des ersten Typs weist zwei konvexe Kopplungsabschnitte 81, 83 mit einer Verjüngung bzw. einem Hals 82 zwischen den konvexen Kopplungsabschnitten auf. Ein Übertragungsglied 90 des zweiten Typs weist zwei konkave Kopplungsabschnitte 91, 93 auf.

Jeder einzelne konvexe Kopplungsabschnitt 81, 83 eines Übertragungsglieds 80 des ersten Typs entspricht hinsichtlich Gestalt und Funktion dem konvexen Kopplungsabschnitt 51, 61 eines Übertragungsglieds 50, 60 des Ausführungsbeispiels aus den Figuren 2 bis 5. Jeder einzelne konkave Kopplungsabschnitt 91, 93 eines Übertragungsglieds 90 des zweiten Typs entspricht hinsichtlich räumlicher Gestalt und Funktion dem konkaven Kopplungsabschnitt 55, 65 eines Übertragungsglieds 50, 60 des Ausführungsbeispiels aus den Figuren 2 bis 5. Insbesondere weist deshalb jedes Übertragungsglied 90 des zweiten Typs zwei Hülsen 70 auf. Die Hülsen entsprechen dem Ausführungsbeispiel der Figuren 2 bis 5.

Die formschlüssige gelenkige Kopplung zwischen einem konvexen Kopplungsabschnitt 81, 83 eines Übertragungsglieds des ersten Typs und einem konkaven Kopplungsabschnitt 91, 93 eines Übertragungsglieds 90 des zweiten Typs entspricht der formschlüssigen mechanischen Kopplung zwischen einem konvexen Kopplungsabschnitt 51 eines Übertragungsglieds und einem konkaven Kopplungsabschnitt 65 eines benachbarten Übertragungsglieds 60 bei dem oben anhand der Figuren 2 bis 5 dargestellten Ausführungsbeispiel. Das in Figur 6 dargestellte Ausführungsbeispiel des flexiblen Abschnitts 46 der Übertragungseinrichtung 40 ermöglicht deshalb ebenso wie das oben anhand der Figuren 2 bis 5 dargestellte Ausführungsbeispiel eine Übertragung von Druck- und Zugkräften sowie von Drehmomenten. Auch die Herstellung des flexiblen Abschnitts 46 gemäß dem in Figur 6 dargestellten Ausführungsbeispiel kann entsprechend dem oben anhand der Figuren 4 und 5 für das Ausführungsbeispiel der Figuren 2 bis 5 dargestellten Verfahren erfolgen.

Figur 7 zeigt ein schematisches Flussdiagram eines Verfahrens zum Herstellen einer Übertragungseinrichtung. Obwohl sich das Verfahren auch zum Herstellen einer Übertragungseinrichtung mit Merkmalen, die sich von den oben anhand der Figuren 1 bis 6 dargestellten unterscheidet, eignet, werden nachfolgend beispielhaft Bezugszeichen aus den Figuren 1 bis 6 verwendet, um das Verständnis zu erleichtern.

Bei einem ersten Schritt 101 werden mehrere Übertragungsglieder 50, 60; 80, 90 mit jeweils zwei Kopplungsabschnitten 51, 61, 55, 65; 81, 83, 91, 93 bereitgestellt. Das Bereitstellen der mehreren Übertragungsglieder umfasst ein Bereitstellen 102 einer Hülse 70 als Bestandteil von einem der Übertragungsglieder 50, 60; 80, 90. Die beim zweiten, Schritt 102 bereitgestellte Hülse 70 weist einen Schlitz 78 auf, der sich vom ersten Ende 71 zum zweiten Ende 72 der Hülse 70 erstreckt. Ferner weist die Hülse 70 einen nach radial innen ragenden Kragen 76 am zweiten Ende 72 der Hülse 70 auf.

Bei einem dritten Schritt 103 wird eine Verjüngung bzw. ein Hals 52; 82 an einem konvexen Kopplungsabschnitt 51; 81, 83 an einem Übertragungsglied 50; 80 vom ersten Ende 71 durch den Schlitz 78 bis zum zweiten Ende 72 der Hülse 70 hindurch geführt. Der dritte Schritt 103 wird beispielsweise, wie oben anhand der Figur 4 dargestellt, durchgeführt.

Bei einem vierten Schritt 104 wird der konvexe Kopplungsabschnitt 51; 81, 83 am ersten Übertragungsglied 50; 80 in den konkaven Kopplungsabschnitt 65; 91, 93 am zweiten Übertragungsglied 60; 90 eingeführt. Dabei wird gleichzeitig der konkave Kopplungsabschnitt 65; 91, 93 am zweiten Übertragungsglied 60; 90 in die Hülse 70 eingeführt. Bei einem fünften Schritt 105 wird die Hülse 70 mit dem zweiten Übertragungsglied 60; 90 gefügt, insbesondere geschweißt, gelötet oder geklebt.

### Bezugszeichen

- 10: medizinisches Instrument
- 11: proximales Ende des medizinischen Instruments 10
- 12: distales Ende des medizinischen Instruments 10
- 14: Schaft des medizinischen Instruments 10
- 16: Gelenk am Schaft 14
- 18: Längsachse des medizinischen Instruments 10
- 20: Handhabungseinrichtung am proximalen Ende 11 des medizinischen Instruments 10
- 21: feststehendes Griffteil der Handhabungseinrichtung 20
- 22: bewegbares Griffteil der Handhabungseinrichtung 20
- 23: Drehrad an der Handhabungseinrichtung 20
- 30: Werkzeug am distalen Ende 12 des medizinischen Instruments 10
- 31: erstes Maulteil am Werkzeug 30
- 32: zweites Maulteil am Werkzeug 30
- 38: Rotationsachse des Werkzeugs 30
- 40: Übertragungseinrichtung
- 46: flexibler Abschnitt der Übertragungseinrichtung 40
- 50: Übertragungsglied
- 51: konvexer Kopplungsabschnitt des Übertragungsglieds 60
- 52: Hals des Übertragungsglieds 50
- 53: Nut am Übertragungsglied 50
- 54: schulterförmiger Bereich am konvexen Kopplungsabschnitt 51
- 55: konkaver Kopplungsabschnitt am Übertragungsglied 50
- 56: Ausnehmung am konkaven Kopplungsabschnitt 55
- 57: Wand um Ausnehmung 56
- 58: Längsachse des Übertragungsglieds 50
- 60: Übertragungsglied
- 61: konvexer Kopplungsabschnitt des Übertragungsglieds 60
- 62: Hals des Übertragungsglieds 60
- 63: Nut am Übertragungsglied 60
- 64: schulterförmiger Bereich am konvexen Kopplungsabschnitt 61
- 65: konkaver Kopplungsabschnitt am Übertragungsglied 60
- 66: Ausnehmung am konkaven Kopplungsabschnitt 65
- 67: Wand um Ausnehmung 66
- 68: Längsachse des Übertragungsglieds 60
- 70: Hülse des Übertragungsglieds 60
- 71: erstes Ende der Hülse 70
- 72: zweites Ende der Hülse 70
- 74: zylindrische Wand der Hülse 70
- 76: Rand am zweiten Ende 72 der Hülse 70
- 78: Schlitz in Hülse 70
- 80: Übertragungsglied eines ersten Typs
- 81: erster konvexer Kopplungsabschnitt des Übertragungsglieds 80
- 82: Hals des Übertragungsglieds 80
- 83: zweiter konvexer Kopplungsabschnitt des Übertragungsglieds 80
- 84: schulterförmiger Bereich am ersten konvexen Kopplungsabschnitt 81
- 88: Längsachse des Übertragungsglieds 80
- 90: Übertragungsglied eines zweiten Typs
- 91: erster konkaver Kopplungsabschnitt des Übertragungsglieds 90
- 93: zweiter konkaver Kopplungsabschnitt des Übertragungsglieds 90
- 96: Ausnehmung am konkaven Kopplungsabschnitt 95
- 97: Wand um Ausnehmung 96
- 98: Längsachse des Übertragungsglieds 90
- 101: erster Schritt (Bereitstellen)
- 102: zweiter Schritt (Bereitstellen)
- 103: dritter Schritt (Hindurchführen)
- 104: vierter Schritt (Einführen)
- 105: fünfter Schritt (Fügen)

## Patentansprüche

1. **Übertragungseinrichtung** (40) zum Übertragen einer Kraft und eines Drehmoments zwischen einer Handhabungseinrichtung (20) und einem Werkzeug (30) eines medizinischen Instruments (10), mit: miteinander gelenkig verbundenen Über**tragungsgliedern** (50, 60; 80, 90), wobei jeweils ein **Kopplungsabschnitt** (51; 81, 83) eines ersten Übertragungsglieds (50; 80) in einem **Kopplungsabschnitt** (65; 91, 93) eines benachbarten zweiten Übertragungsglieds (60; 90) derart formschlüssig gehalten ist, dass eine **Rotation** des ersten Übertragungsglieds (50; 80) um seine Längsachse (58; 88) eine Rotation des zweiten Übertragungsglieds (60; 90) um seine Längsachse (68; 98) bewirkt,
der eine Kopplungsabschnitt (51; 81, 82) konvex und der andere **Kopplungsabschnitt** (65; 91, 93) **konkav ist,**
**dadurch gekennzeichnet, dass**
jeweils ein konvexer Kopplungsabschnitt (51; 81, 82) eines ersten Übertragungsglieds (50; 80) in einem konkaven Kopplungsabschnitt (65; 91, 93) eines benachbarten zweiten Übertragungsglieds (60; 90) derart formschlüssig gehalten ist, dass eine **Zugkraft** zwischen dem ersten Übertragungsglied (50; 80) und dem zweiten Übertragungsglied (60; 90) übertragbar ist,
bei der am konvexen Kopplungsabschnitt (51; 81, 83) eines ersten Übertragungsglieds (50; 80) ein erster **Haltebereich** (54; 84) und am konkaven Kopplungsabschnitt (65; 91, 93) eines benachbarten zweiten Übertragungsglieds (60; 90) ein zweiter **Haltebereich** (76) vorgesehen sind, der erste Haltebereich (54; 84) und der zweite Haltebereich (76) formschlüssig miteinander verbunden sind, um eine Zugkraft zwischen dem ersten Übertragungsglied (50; 80) und dem zweiten Übertragungsglied (60; 90) aufzunehmen, und
bei der bezogen auf eine Schnittebene, die die Längsachse (58; 88) des ersten Übertragungsglieds (50; 80) enthält, der **erste Haltebereich** (54; 84) zu einem **konka**ven Bereich (52; 82) der Kontur des Übertragungsglieds (50; 80) benachbart ist, der **zweite Haltebereich** (76) eine im Wesentlichen **konvexe** Kontur aufweist, und
bei der der erste Haltebereich (54; 85) am ersten Übertragungsglied (50; 80) durch den Rand einer **Nut** (53) gebildet wird, der zweite Haltebereich (76) am zweiten Übertragungsglied (60; 90) die Gestalt eines **Kragens,** der in die Nut (53) am ersten Übertragungsglied (50; 80) eingreift, aufweist und
bei der eine **Hülse** (70), die den konkaven Kopplungsabschnitt (65) umgibt, den nach radial innen ragenden Kragen (76) bildet

2. Übertragungseinrichtung (40) nach dem vorangehenden Anspruch, bei der die Hülse (70) einen **Schlitz** (78) umfasst, der parallel zur Längsachse (68; 98) des zweiten Übertragungsglieds (60; 90) ist.

3. Übertragungseinrichtung (40) nach dem vorangehenden Anspruch, bei der der erste Haltebereich (54; 84) am ersten Übertragungsglied (50; 80) durch eine Verjüngung (52; 82) des ersten Übertragungsglieds (50; 80) an einer von dem zweiten Übertragungsglied (60; 90) abgewandten Seite des konvexen Kupplungsabschnitts (51; 81, 83) gebildet ist, der Schlitz (78) in der Hülse (70) eine **Breite** aufweist, die nicht kleiner ist als eine minimale Breite der Verjüngung (52; 82).

4. **Werkzeug** (30) für ein medizinisches Instrument (10) mit einer Übertragungseinrichtung (40) nach einem der vorangehenden Ansprüche.

5. **Medizinisches Instrument** (10) mit einer Übertragungseinrichtung (40) nach einem der Ansprüche 1 bis 3.

6. **Verfahren zum Herstellen** einer Übertragungseinrichtung (40) nach einem der Ansprüche 1 bis 3, mit folgenden Schritten: Bereitstellen (101) mehrerer **Übertragungsglieder** (50, 60; 80, 90) mit jeweils zwei **Kopplungsabschnitten** (51, 61, 65; 81, 83, 91, 93), wobei eines der mehreren Übertragungsglieder (50, 60; 80, 90) eine Hülse (70) mit einem sich vom ersten Ende (71) zum zweiten Ende (72) der Hülse (70) erstreckenden **Schlitz** (78) und einem nach radial innen ragenden Kragen (76) am zweiten Ende (72) der Hülse (70) aufweist; Hindurchführen (103) einer **Verjüngung** (52; 82) an einem konvexen Kopplungsabschnitt (51; 81, 83) an einem ersten Übertragungsglied (50; 80) vom ersten Ende (71) durch den **Schlitz** (78) bis zum zweiten Ende (72) der Hülse (70); **Einführen** (104) eines konkaven Kopplungsabschnitts (65; 91, 93) an einem zweiten Übertragungsglied (60; 90) in die Hülse (70), wobei der konvexe Kopplungsabschnitt (51; 81, 83) am ersten Übertragungsglied (50; 80) in den konkaven Kopplungsabschnitt (65; 91, 93) am zweiten Übertragungsglied (60; 90) eingeführt wird; **Fügen** (105) der Hülse (70) mit dem zweiten Übertragungsglied (60; 90).

## Claims

1. Transmission device (40) for transmitting a force and a torque between a handling device (20) and a tool (30) of a medical instrument (10), said transmission device comprising: transmission members (50, 60; 80, 90) interconnected in a hinged manner, wherein a coupling portion (51; 81, 83) of a first transmission member (50; 80) is in each case held interlockingly in a coupling portion (65; 91, 93) of an adjacent second transmission member (60; 90) in such a way that a rotation of the first transmission member (50; 80) about its longitudinal axis (58; 88) causes a rotation of the second transmission member (60; 90) about its longitudinal axis (68; 98),
the one coupling portion (51; 81, 82) is convex and the other coupling portion (65; 91, 93) is concave,
**characterized in that**
a convex coupling portion (51; 81, 82) of a first transmission member (50; 80) is in each case held interlockingly in a concave coupling portion (65; 91, 93) of an adjacent second transmission member (60; 90) in such a way that a tensile force can be transmitted between the first transmission member (50; 80) and the second transmission member (60; 90),
wherein a first retaining region (54; 84) is provided on the convex coupling portion (51; 81, 83) of a first transmission member (50; 80) and a second retaining region (76) is provided on the concave coupling portion (65; 91, 93) of an adjacent second transmission member (60; 90), and the first retaining region (54; 84) and the second retaining region (76) are interlockingly interconnected so as to take up a tensile force between the first transmission member (50; 80) and the second transmission member (60; 90), and
wherein, relative to a sectional plane containing the longitudinal axis (58; 88) of the first transmission member (50; 80), the first retaining region (54; 84) is adjacent to a concave region (52; 82) of the contour of the transmission member (50; 80), and the second retaining region (76) has a substantially convex contour, and
wherein the first retaining region (54; 85) on the first transmission member (50; 80) is formed by the edge of a groove (53), and the second retaining region (76) on the second transmission member (60; 90) has the form of a collar, which engages in the groove (53) on the first transmission member (50; 80) and
wherein a sleeve (70), which surrounds the concave coupling portion (65), forms the radially inwardly protruding collar (76).

2. Transmission device (40) according to the preceding claim, wherein the sleeve (70) comprises a slit (78), which is parallel to the longitudinal axis (68; 98) of the second transmission member (60; 90).

3. Transmission device (40) according to the preceding claim, wherein the first retaining region (54; 84) on the first transmission member (50; 80) is formed by a taper (52; 82) of the first transmission member (50; 80) on a side, facing away from the second transmission member (60; 90), of the convex coupling portion (51; 81, 83), and the slit (78) in the sleeve (70) has a width that is no smaller than a minimum width of the taper (52; 82).

4. Tool (30) for a medical instrument (10) comprising a transmission device (40) according to one of the preceding claims.

5. Medical instrument (10) comprising a transmission device (40) according to one of Claims 1 to 3.

6. Method for producing a transmission device (40) according to one of Claims 1 to 3, said method comprising the following steps: provision (101) of a plurality of transmission members (50, 60; 80, 90) each having two coupling portions (51, 61, 65; 81, 83, 91, 93), wherein one of the plurality of transmission members (50, 60; 80, 90) has a sleeve (70) with a slit (78) extending from the first end (71) to the second end (72) of the sleeve (70) and with a radially inwardly protruding collar (76) at the second end (72) of the sleeve (70); guidance (103) of a taper (52; 82) on a convex coupling portion (51; 81, 83) on a first transmission member (50; 80) from the first end (71) through the slit (78) as far as the second end (72) of the sleeve (70); introduction (104) of a concave coupling portion (65; 91, 93) on a second transmission member (60; 90) into the sleeve (70), wherein the convex coupling portion (51; 81, 83) on the first transmission member (50; 80) is introduced into the concave coupling portion (65; 91, 93) on the second transmission member (60; 90); joining (105) of the sleeve (70) to the second transmission member (60; 90).

## Revendications

1. Dispositif de transfert (40) pour le transfert d'une force et d'un couple entre un dispositif de manipulation (20) et un outil (30) d'un instrument médical (10), comprenant : des organes de transfert (50, 60 ; 80, 90) connectés de manière articulée les uns aux autres, une portion d'accouplement (51 ; 81, 83) d'un premier organe de transfert (50 ; 80) étant retenue à chaque fois par engagement par correspondance de formes dans une portion d'accouplement (65 ; 91, 93) d'un deuxième organe de transfert adjacent (60 ; 90) de telle sorte qu'une rotation du premier organe de transfert (50 ; 80) autour de son axe longitudinal (58 ; 88) provoque une rotation du deuxième organe de transfert (60 ; 90) autour de son axe longitudinal (68 ; 98),
l'une des portions d'accouplement (51 ; 81, 82) étant convexe et l'autre portion d'accouplement (65 ; 91, 93) étant concave,
**caractérisé en ce que**
une portion d'accouplement convexe (51 ; 81, 82) d'un premier organe de transfert (50 ; 80) étant à chaque fois retenue par engagement par correspondance de formes dans une portion d'accouplement concave (65 ; 91, 93) d'un deuxième organe de transfert adjacent (60 ; 90) de telle sorte qu'une force de traction entre le premier organe de transfert (50 ; 80) et le deuxième organe de transfert (60 ; 90) puisse être transmise,
une première région de retenue (54 ; 84) étant prévue au niveau de la portion d'accouplement convexe (51 ; 81, 83) d'un premier organe de transfert (50 ; 80) et une deuxième région de retenue (76) étant prévue au niveau de la portion d'accouplement concave (65 ; 91, 93) d'un deuxième organe de transfert adjacent (60 ; 90), la première région de retenue (54 ; 84) et la deuxième région de retenue (76) étant connectées l'une à l'autre par engagement par correspondance de formes afin de recevoir une force de traction entre le premier organe de transfert (50 ; 80) et le deuxième organe de transfert (60 ; 90), et
la première région de retenue (54 ; 84) étant adjacente, par rapport à un plan de coupe qui contient l'axe longitudinal (58 ; 88) du premier organe de transfert (50 ; 80), à une région concave (52 ; 82) du contour de l'organe de transfert (50 ; 80), la deuxième région de retenue (76) présentant un contour essentiellement convexe, et
la première région de retenue (54 ; 85) étant formée au niveau du premier organe de transfert (50 ; 80) par le bord d'une rainure (53), la deuxième région de retenue (76) présentant, au niveau du deuxième organe de transfert (60 ; 90), la forme d'un col qui s'engage dans la rainure (53) au niveau du premier organe de transfert (50 ; 80), et
une douille (70) qui entoure la portion d'accouplement concave (65) formant le col (76) saillant radialement vers l'intérieur.

2. Dispositif de transfert (40) selon la revendication précédente, dans lequel la douille (70) comprend une fente (78) qui est parallèle à l'axe longitudinal (68 ; 98) du deuxième organe de transfert (60 ; 90).

3. Dispositif de transfert (40) selon la revendication précédente, dans lequel la première région de retenue (54 ; 84) au niveau du premier organe de transfert (50 ; 80) est formée par un rétrécissement (52 ; 82) du premier organe de transfert (50 ; 80) au niveau d'un côté de la portion d'accouplement convexe (51 ; 81, 83) opposé au deuxième organe de transfert (60 ; 90), et la fente (78) dans la douille (70) présente une largeur qui n'est pas inférieure à une largeur minimale du rétrécissement (52 ; 82).

4. Outil (30) pour un instrument médical (10) comprenant un dispositif de transfert (40) selon l'une quelconque des revendications précédentes.

5. Instrument médical (10) comprenant un dispositif de transfert (40) selon l'une quelconque des revendications 1 à 3.

6. Procédé de fabrication d'un dispositif de transfert (40) selon l'une quelconque des revendications 1 à 3, comprenant les étapes suivantes : fourniture (101) de plusieurs organes de transfert (50, 60 ; 80, 90) comprenant à chaque fois deux portions d'accouplement (51, 61, 65 ; 81, 83, 91, 93), l'un de la pluralité d'organes de transfert (50, 60 ; 80, 90) présentant une douille (70) avec une fente (78) s'étendant depuis la première extrémité (71) jusqu'à la deuxième extrémité (72) de la douille (70) et un col (76) faisant saillie radialement vers l'intérieur au niveau de la deuxième extrémité (72) de la douille (70) ; guidage (103) d'un rétrécissement (52 ; 82) au niveau d'une portion d'accouplement convexe (51 ; 81, 83) au niveau d'un premier organe de transfert (50 ; 80) depuis la première extrémité (71) à travers la fente (78) jusqu'à la deuxième extrémité (72) de la douille (70) ; insertion (104) d'une portion d'accouplement concave (65 ; 91, 93) au niveau d'un deuxième organe de transfert (60 ; 90) dans la douille (70), la portion d'accouplement convexe (51 ; 81, 83) au niveau du premier organe de transfert (50 ; 80) étant insérée dans la portion d'accouplement concave (65 ; 91, 93) au niveau du deuxième organe de transfert (60 ; 90) ; assemblage (105) de la douille (70) au deuxième organe de transfert (60 ; 90).
